# EUROPEAN PATENT APPLICATION

(11) **EP 4 435 101 A1**
(43) Date of publication of application: **25.09.2024**
(21) Application number: 22894779.2
(22) Date of filing: 15.11.2022
(51) Int. Cl.: C12N 15/10, C07K 14/705, A61K 48/00

(54) **MEMBRANE SURFACE PROTEIN CONTAINING GPI ANCHOR REGION**

(30) Priority: 17.11.2021 CN 202111361248
(71) Applicant: Shanghai Juncell Therapeutics Co., Ltd., Shanghai 201800 (CN)
(72) Inventor: JIN, Huajun, Shanghai 201800 (CN); ZHOU, Ying, Shanghai 201800 (CN); WANG, Chao, Shanghai 201800 (CN); HUANG, Chen, Shanghai 201800 (CN)
(74) Representative: Nederlandsch Octrooibureau
(86) International application number: PCT/CN2022/131974
(87) International publication number: WO 2023/088246

(57) **Abstract**

Provided is a membrane surface protein containing a GPI anchor region. Specifically, provided are a fusion protein comprising a functional domain and a GPI anchor region, and the use thereof. The fusion protein can effectively activate and proliferate immune cells, and improve the effector function of immune cells.

## Description

### FIELD OF THE INVENTION

The present description relates to the field of biotechnology, and in particular to a membrane-bound protein containing a GPI anchor region.

### BACKGROUND

Transmembrane proteins are usually anchored to the membrane surface through transmembrane domains, i.e., transmembrane polypeptides. However, in addition to common polypeptide transmembrane domains, proteins can also be localized to the membrane surface through post-translational lipid modification. Most lipid modifications occur in the cytoplasm and help to localize proteins to the membrane, sometimes to specific membrane microdomains. Another type of lipid modification, glycosylphosphatidylinositol (GPI) modification, occurs at the luminal side of the endoplasmic reticulum membrane. The core structure of GPI is synthesized on the endoplasmic reticulum membrane before it is transferred to the precursor protein that has just completed endoplasmic reticulum translocation (ER translocation) to form a nascent GPI-anchored protein. The nascent GPI-anchored protein undergoes several further modifications in the endoplasmic reticulum and Golgi apparatus to form a mature GPI-anchored protein, which is finally transferred to the cell membrane surface and becomes a membrane-bound protein integrated to the cell membrane lipid bilayer through the GPI structure.

The GPI moiety of GPI-anchored proteins consists of a conserved core glycan, phosphatidylinositol, and glycan branch chains. The core structure of glycan is EtNP-6Manα2-Manα6-(EtNP)2Manα4-G1Nα6-inositol-P-lipid. The protein moiety is basically a hydrophobic polypeptide lacking the transmembrane region, with the GPI covalently linked to the C-terminus anchored on the cell membrane. For molecules that function in the form of dimers or multimers, such as cytokines, antibodies, etc., their functionality may be enhanced if they are anchored to the membrane via the structure of GPI-anchored proteins.

### SUMMARY

The present description provides a fusion protein, which comprises a functional domain and a GPI anchor region.

In one or more embodiments, the functional domain functions through dimerization or multimerization.

In one or more embodiments, the functional domain comprises: an antibody or an antigen-binding fragment thereof, and/or an amino acid-based cytokine or a functional fragment thereof.

In one or more embodiments, the cytokines include interleukins or their receptors, chemokines or their receptors, interferons, and TNF-α.

In one or more embodiments, the interleukin or receptor thereof comprises one or more selected from the group consisting of: IL-2, IL-4, IL-6, IL-7, IL-8, IL- 10, IL-12, IL-15, CD25, IL-7R, IL-12Rβ1, IL-12Rβ2 and IL-15R.

In one or more embodiments, the chemokine or receptor thereof comprises one or more selected from the group consisting of: CXCL2, CXCL3, CXCL4, CXCL5, CXCL6, CXCL7, CXCL8, CXCL9, CXCL10, CXCL11, CXCL12, CXCL13, CXCL14, CCL1, CCL2, CCL3, CCL4, CCL5, CCL7, CCL8, CCL11, CCL13, CCL14, CCL15, CCL17, CCL18, CCL19, CCL20, CCL21, CCL22, CCL23, CCL24, CCL25, CCL26, CCL27, CCL28, CXCR1, CXCR2, CXCR3A, CXCR3B, CXCR4, CXCR5, CXCR6, CCR2, CCR5, CCR1, CCR3, CCR4, CCR6, CCR7, CCR8, CCR9, CCR10 and XCR1.

In one or more embodiments, the interferon is IFN-γ.

In one or more embodiments, the antibodies include blocking antibodies and activating antibodies.

In one or more embodiments, the antibody is an antibody that targets a tumor-associated antigen, such as an antibody that targets HER2, HER3, CD137, or PD-1.

In one or more embodiments, the GPI anchor region comprises one or more molecules selected from the group consisting of: CD44, CD56, CD73, CD55, Thyl, AchE, IAP, ALPP, CD59, CD14, CD16, CD24, CD28, CD48, CD52, CD58, CD66a, CD66c, CD66d, CD66e, CD67, CD87, CD108, CD157, uPAR, JMH protein, GDNFR, CNTFR, TAG-1, PrP, phosphatidylinositol protein, semaphorin 7, CEA, GFR, Ly6G, transferrin receptor, contactin (F3) and T-cadherin, or GPI anchor domains thereof.

In one or more embodiments, the GPI anchor region comprises one or more selected from the group consisting of: CD52, CD48, CD55, ALPP, and CD90 proteins, or GPI anchor domains thereof.

In one or more embodiments, the GPI anchor region comprises the sequence set forth in positions 232-268 of SEQ ID NO:2, or variants having at least 90% sequence identity thereto and being able to form GPI anchoring structures.

In one or more embodiments, the functional domain is located at the N-terminal to the GPI anchor region.

In one or more embodiments, the fusion protein optionally further comprises a spacer between the functional domain and the GPI anchor region.

In one or more embodiments, the spacer has a length of 12-66aa, such as 12aa, 54aa, 55aa or 66aa.

In one or more embodiments, the spacer comprises: a linker and/or a protein extracellular domain.

In one or more embodiments, the linker comprises the sequence set forth in positions 177-188 of SEQ ID NO: 4, or a sequence having at least 90% sequence identity thereto.

In one or more embodiments, the protein extracellular domain is a CD8 extracellular domain and/or a BCMA extracellular domain.

In one or more embodiments, the protein extracellular region comprises the sequence shown in positions 177-231 of SEQ ID NO:2 or positions 189-242 of SEQ ID NO:4, or a sequence having at least 90% sequence identity thereto.

In one or more embodiments, the fusion protein further comprises a signal peptide.

In one or more embodiments, the signal peptide is the signal peptide of the protein that the GPI anchor region derived from.

In one or more embodiments, the signal peptide is a signal peptide of any one or more protein selected from the group consisting of: CD52, CD48, CD55, ALPP, and CD90.The description also provides a nucleic acid molecule comprising a sequence selected from the group consisting of:
(1) the coding sequence of the fusion protein described in the first aspect of the present description or its fragment function as an amplification primer or detection probe,
(2) a variant with at least 80% sequence identity to (1),
(3) a complementary sequence of (1) or (2).

Another aspect of the description provides a nucleic acid construct comprising the nucleic acid molecule according to any embodiment of the present description.

In one or more embodiments, the nucleic acid construct is a cloning vector, expression vector, or integration vector.

Another aspect of the description provides a host cell, the host cell:
(1) contains, expresses and/or secrets the fusion protein according to any embodiment of the first aspect of the present description, and/or
(2) comprises any one or more of the nucleic acid molecules and nucleic acid constructs described in any embodiment of the present description.

In one or more embodiments, the host cell is a lymphocyte, such as a T cell, NK cell, TIL, etc.

In one or more embodiments, the lymphocytes are T cells, NK cells, or tumor-infiltrating lymphocytes (TIL).

In one or more embodiments, the T cells or TILs further express exogenous TCR and/or CAR.

In one or more embodiments, the T cells include: CAR-T cells, TCR-T cells.

The present description also provides a method for preparing the fusion protein according to any embodiment of the first aspect of the present description, comprising incubating the host cell according to any embodiment of the present description.

Another aspect of the present description provides a pharmaceutical composition, comprising any one or more of the fusion proteins, nucleic acid molecules, nucleic acid constructs and cells described in any embodiment of the present description, and pharmaceutically acceptable excipients.

The present description also provides the use of any one or more of the fusion proteins, nucleic acid molecules, nucleic acid constructs, cells and pharmaceutical compositions described in any embodiment of the present description in the manufacture of an antitumor medicament.

The present description also provides a method for treating and/or preventing tumors, comprising administering to a subject in need thereof a therapeutically and/or prophylactically effective amount of any one or more of the fusion proteins, the fusion protein, the nucleic acid molecule, the nucleic acid construct and the cell described in any embodiment of the present description.

### Advantages of the invention:

1) Anchoring cytokines or antibodies on the surface of immune cells through GPI-anchored proteins can effectively activate and proliferate immune cells and enhance the effector function of immune cells;
2) The expression of foreign proteins such as cytokines and antibodies that are anchored to the cell membrane surface through GPI-anchored proteins is significantly improved compared to that of those proteins bound through conventional amino acid transmembrane domains;
3) Compared with the GPI signal sequence disclosed in CN110709416A, the CD52 used in the present description is an intact protein, and the CD52-anchored membrane surface protein of the present description does not introduce potential new antigen sequences, has low immunogenicity, and is more conducive to subsequent *in vivo* applications.

### BRIEF DESCRIPTION OF DRAWINGS

Fig 1: Brightfield microscopic morphology of T cells expressing cytokines bound to the membrane surface through a GPI anchor region and T cells expressing EGFP .
Fig 2: Number of T cells expressing cytokines bound to the membrane surface through a GPI anchor region.
Fig 3: Viability of T cells expressing cytokines bound to the membrane surface through a GPI anchor region.
Fig 4: Flow cytometric detection results of T cells expressing cytokines bound to the membrane surface through a GPI anchor region.
Fig 5: Cytokine secretion levels of T cells expressing cytokines bound to the membrane surface through a GPI anchor region.
Fig 6: Flow cytometry results of T cells expressing cytokines bound to the membrane surface through a GPI anchor region and a TCR.
Fig 7: T cells expressing cytokines bound to the membrane surface through the GPI anchor region and a TCR have a killing effect on target cells at the effector-to-target ratio of 1:1.
Fig 8: T cells expressing cytokines bound to the membrane surface through the GPI anchor region and a TCR have a killing effect on target cells at the effector-to-target ratio of 0.5:1.
Fig 9: The killing effect of TILs expressing HER2 antibodies bound to the membrane surface through a GPI anchor region on patient-matched tumor primary cells.
Fig 10: The killing effect of T cells expressing HER2 antibodies bound to the membrane surface through a GPI anchor region and a TCR on target cells.
Fig 11: Number of T cells expressing cytokines anchored to the membrane surface through different GPI proteins.
Fig 12: Viability of T cells expressing cytokines anchored to the membrane surface through different GPI proteins.
Fig 13: The killing effect of T cells expressing cytokines anchored on the membrane surface through different GPI proteins and a TCR and TCR-T cells on target cells at the effector-to-target ratio of 0.5:1.
Fig 14: Tumor volume changes in tumor-bearing mice administered in vivo with TCR-T cells and TCR-T cells expressing membrane surface cytokines anchored by GPI proteins.
Fig 15: Changes in tumor fluorescence values in tumor-bearing mice administered *in vivo* with TCR-T and TCR-T cells expressing membrane surface cytokines anchored by GPI proteins.
Fig 16: Fluorescent intravital imaging of tumors in tumor-bearing mice administered *in vivo* with TCR-T and TCR-T cells expressing membrane surface cytokines anchored by GPI proteins.
Fig 17: Number of human lymphocytes in the peripheral blood of tumor-bearing mice administered *in vivo* with TCR-T and TCR-T cells expressing membrane surface cytokines anchored by GPI proteins.

### DETAILED DESCRIPTION

The practice of the present description will employ, unless otherwise indicated, conventional techniques of molecular biology (including recombinant techniques), microbiology, cell biology, biochemistry and immunology, which are within the skill of the art. Such techniques are explained fully in the literature, such as, Molecular Cloning: A Laboratory Manual, second edition (Sambrook et al., 1989); Oligonucleotide Synthesis (MJ. Gait, ed., 1984); Animal Cell Culture (RJ. Freshney, ed., 1987); Methods in Enzymology (Academic Press, Inc.); Current Protocols in Molecular Biology (F.M. Ausubel et al., eds 1987, and periodic updates); PCR: The Polymerase Chain Reaction, (Mullis et al., ed., 1994); A Practical Guide to Molecular Cloning (Perbal Bernard V., 1988); Phage Display: A Laboratory Manual (Barbas et al., 2001).

GPI-anchored proteins are connected to lipid rafts of the cell membrane through interactions between lipids or polysaccharides. The inventor has discovered through research that for molecules that need to function through dimerization or multimerization such as cytokines, antibodies, etc., anchoring them on the membrane surface through the structure of GPI-anchored proteins can improve their own functions, for example, effectively enhancing the proliferation and activation of immune cells and the killing effect on target cells.

The present description first provides a fusion protein, which comprises a functional domain and a GPI anchor region. The GPI anchor region is used to anchor functional domains to the surface of cells (such as immune cells) to promote dimerization or multimerization of the functional domains. The functional domain is preferably located adjacent to the N-terminus of the GPI anchor region.

The GPI anchor region comprises one or more molecules selected from the group consisting of: CD44, CD56, CD73, CD55, Thyl, AchE, IAP, ALPP, CD59, CD14, CD16, CD24, CD28, CD48, CD52, CD58, CD66a, CD66c, CD66d, CD66e, CD67, CD87, CD108, CD157, uPAR, JMH protein, GDNFR, CNTFR, TAG-1, PrP, phosphatidylinositol protein, semaphorin 7, CEA, GFR, Ly6G, transferrin receptor, contactin (F3) and T-cadherin, or a GPI anchoring domain thereof (also referred to herein as a GPI signal sequence); preferably, the GPI anchor region is CD52 protein or its GPI anchoring domain. GPI-anchored proteins or their anchoring domain sequences are known in the art. Moreover, those skilled in the art can easily obtain the anchoring domain sequence of the GPI-anchored protein based on its sequence.

In one or more embodiments, the GPI anchor region comprises the sequence shown in positions 232-268 of SEQ ID NO: 2, or a variant having at least 90% sequence identity thereto and having the function of forming a bond to GPI. Alternatively, the GPI anchor region may be a GPI domain of CD48, a GPI domain of CD55, a GPI domain of ALPP and a GPI domain of CD90, the amino acid sequences of which are respectively shown in SEQ ID NO: 15-18 described on page 14 in the specification of CN110709416A.

As used herein, functional domains include any polypeptide or protein that functions through dimerization or multimerization. In some embodiments, the functional domain comprises a cytokine or functional fragment thereof.

Cytokines that can be used as functional domains described herein are mainly polypeptide cytokines based on amino acids, including but not limited to: interleukins or their receptors, chemokines or their receptors, interferons and TNF-α. The interleukin or its receptor comprises one or more selected from the group consisting of: IL-2, IL-4, IL-6, IL-7, IL-8, IL-10, IL-12, IL-15, CD25, IL-7R, IL-12Rβ1, IL-12Rβ2 and IL-15R. The chemokine or its receptor comprises one or more selected from the group consisting of: CXCL2, CXCL3, CXCL4, CXCL5, CXCL6, CXCL7, CXCL8, CXCL9, CXCL10, CXCL11, CXCL12, CXCL13, CXCL14, CCL1, CCL2, CCL3, CCL4, CCL5, CCL7, CCL8, CCL11, CCL13, CCL14, CCL15, CCL17, CCL18, CCL19, CCL20, CCL21, CCL22, CCL23, CCL24, CCL25, CCL26, CCL27, CCL28, CXCR1, CXCR2, CXCR3A, CXCR3B, CXCR4, CXCR5, CXCR6, CCR2, CCR5, CCR1, CCR3, CCR4 , CCR6, CCR7, CCR8, CCR9, CCR10 and XCR1. The interferon is, for example, IFN-γ. The above-mentioned cytokines include any known forms thereof, such as subtypes and derivatives. The amino acid sequences of these cytokines are within the routine knowledge of those skilled in the art.

The functional domain may also be an antibody or an antigen-binding fragment thereof. As used herein, "antibody" in a broad sense can be any type of specific binding molecule directed against an antigen, such as full-length antibodies (which have an immunoglobulin Fc region), heavy chain antibodies, nanobodies, minibodies, affibodies, target binding regions of receptors, cell adhesion molecules, ligands, etc. In some embodiments, antibodies include monoclonal antibodies, antibody compositions with multiple epitope specificities, multispecific antibodies (e.g., bispecific antibodies), diabodies, and single chain molecules, and antibody fragments, in particular, antigen-binding fragments such as Fab, F(ab')2 and Fv. The antibodies include blocking antibodies and activating antibodies. In tumor-related embodiments, the antibody is preferably an antibody targeting a tumor-associated antigen, such as an antibody targeting HER2, HER3, CD137 or PD-1. Exemplarily, the HER2-targeting antibody is a single-chain antibody, preferably having the sequence shown in positions 25-268 of SEQ ID NO: 8. Or, exemplarily, the CD137-targeting antibody is a single-chain antibody, preferably having the sequence shown in positions 25-269 of SEQ ID NO: 10.

The fusion protein also optionally comprises a spacer between the functional domain and the GPI anchor region. Whether or not to use a spacer is a routine choice for those skilled in the art and therefore the spacer does not necessarily exist. Usually, the length of the spacer is 10-70aa, preferably 12-66aa, such as 12aa, 54aa, 55aa or 66aa or a range defined by any two of the foregoing values. The spacer may include a linker, such as a polypeptide sequence consisting of G and/or S, such as a polypeptide of GS, GSG, GSSG, SGS, or GGGGS repeated by n times (e.g., 3 times). Exemplary linkers have the sequence set forth in positions 177-188 of SEQ ID NO: 4, or a sequence that has at least 90% sequence identity thereto. The spacer may contain the extracellular domain of some proteins. For example, CD8 extracellular domain and BCMA extracellular domain. Exemplary linker protein extracellular regions have the sequence shown in positions 177-231 of SEQ ID NO: 2 or positions 189-242 of SEQ ID NO: 4, or a sequence having at least 90% sequence identity thereto.

Fusion proteins of the description can be modified to affect function. The present description comprises fusion proteins with modified glycosylation patterns. Alternatively, modifications can be made to remove undesired glycosylation sites. The fusion protein may also have modifications that facilitate expression, secretion, and purification, such as signal peptides. In one or more embodiments, the signal peptide is the signal peptide of the protein that the GPI anchor region is derived from. Exemplarily, the fusion protein herein comprises any one or more signal peptides selected from the group consisting of: the signal peptides of CD52, CD48, CD55, ALPP, and CD90 protein.

In specific embodiments, the fusion protein of the present description comprises a structure, from N-terminus to C-terminus, selected from the group consisting of: (1) IL-7, CD8 extracellular region, CD52, and optionally a CD52 signal peptide at N-terminus, (2) IL-7, a linker, BCMA extracellular domain, CD52, and optionally a CD52 signal peptide at N-terminus, (3) Her2 antibody, a linker, CD52, and optionally a CD52 signal peptide at N-terminus, (4) CD137 antibody, a linker, CD52, and optionally a CD52 signal peptide at N-terminus, (5) IL-7, CD8 extracellular region, CD48 GPI anchoring domain, and optionally has a CD48 signal peptide at N-terminus, (6) IL-7, CD8 extracellular region, CD55 GPI anchoring domain , and optionally a CD55 signal peptide at N-terminus, (7) IL-7, CD8 extracellular region, ALPP GPI anchoring domain, and optionally an ALPP signal peptide at N-terminus, (8) IL-7, CD8 extracellular region, CD90 GPI anchoring domain, and optionally a CD90 signal peptide at N-terminus.

The fusion protein of the present description also comprises variants thereof that have the same function. These variants include (but are not limited to): deletion, insertion and/or substitution of several (usually 1-50, preferably 1-30, 1-20, 1-10, 1-8, 1-5) amino acids, and addition or deletion of one or several (usually within 20, preferably within 10, more preferably within 5) amino acids at the C-terminus and/or N-terminus. For example, in the art, substitution with amino acids of approaching or similar property generally does not alter the function of a protein. In the art, amino acids with similar property tend to refer to families of amino acids with similar side chains, which have been well defined in the art. These families include amino acids with basic side chains (e.g., lysine, arginine, histidine), amino acids with acidic side chains (e.g., aspartic acid, glutamic acid), amino acids with uncharged polar side chains (e.g., glycine, asparagine, glutamine, serine, threonine, tyrosine, cysteine), amino acids with non-polar side chains (e.g. alanine, valine, leucine, isoleucine, lactic acid, phenylalanine, methionine, tryptophan), amino acids with beta-branched side chains (e.g., threonine, valine, isoleucine), and amino acids with aromatic side chains (e.g., tyrosine, phenylalanine, tryptophan, histidine). For another example, the addition of one or more amino acids to the amino terminus and/or carboxy terminus also generally does not alter the function of a polypeptide or protein. The conservative amino acid substitutions of many commonly known non-genetic coding amino acids are known in the art. Conservative substitutions of other non-coding amino acids can be determined based on a comparison of their physical properties with those of the amino acids that are genetically encoded.

Any peptide having high homology (for example, 70% or higher homology to the sequence shown in SEQ ID NO: 2 or 4; preferably, 80% or higher homology; more preferably, 90% or higher homology, such as 95%, 98% or 99% homology) to the fusion protein, and having similar or identical functions to the fusion protein are also included in the present description. The "similar or identical function" mainly refers to the function of the functional domain of the fusion protein (such as anti-tumor) and the function of the GPI anchor region (that is, forming a bond to GPI to anchor on the membrane).

The present description also comprises analogs of the claimed fusion proteins. The difference between these analogs and the original fusion protein may be differences in amino acid sequences, in modifications that do not affect the sequences, or both. Analogues of these proteins include natural or induced genetic variants. Induced variants can be obtained by various techniques, such as random mutagenesis by radiation or exposure to mutagens, site-directed mutagenesis or other techniques known in molecular biology. Analogues also include those with residues that differ from natural L-amino acids (e.g., D-amino acids), as well as analogs with non-naturally occurring or synthetic amino acids (e.g., β, γ-amino acids). It should be understood that the proteins of the present description are not limited to the representative proteins exemplified above.

The polypeptide fragments, derivatives or analogs of the description may also be: (i) a polypeptide formed by fusion of a mature polypeptide with another compound (such as a compound that extends the half-life of the polypeptide, such as polyethylene glycol); or (ii) an additional polypeptide formed by fusing an amino acid sequence to the polypeptide sequence (such as a leader sequence or secretion sequence or a sequence used to purify the polypeptide or an original protein sequence). According to the teaching herein, these fragments, derivatives, and analogues belong to the common knowledge to those skilled in the art.

The present description also relates to polynucleotide sequences encoding the fusion proteins of the present description or variants, analogs, and derivatives thereof, and nucleic acid molecules comprising the sequences. The polynucleotide may be in the form of DNA or RNA. DNAs include cDNAs, genomic DNAs, or synthetic DNAs. DNAs can be single-stranded or double-stranded. DNAs can be coding or noncoding strand.

The present description also relates to variants of the above-mentioned nucleic acid molecules, which encode fusion proteins or fragments, analogs and derivatives thereof having the same amino acid sequence as the present description. The variants may be naturally occurring allelic variants or non-naturally occurring variants. These nucleotide variants include substitution variants, deletion variants, and insertion variants. As is known in the art, an allelic variant is an alternative form of a polynucleotide, which may be the substitution, deletion, or insertion of one or more nucleotides, but does not substantially alter the function of the polypeptide it encodes. As used herein, degenerate variants in the present description refer to nucleic acid sequences that encode the fusion protein but have different coding region sequences. "Polynucleotide encoding a polypeptide" may include a polynucleotide encoding the polypeptide, or may include a polynucleotide further including additional coding and/or non-coding sequences.

The present description also relates to polynucleotides that hybridize (complementary) to the above-mentioned sequences and have at least 50%, preferably at least 70%, and more preferably at least 80% identity between the two sequences. The description particularly relates to polynucleotides that can hybridize with the polynucleotides of the description under strict conditions. In the present description, "strict conditions" refer to: (1) hybridization and elution at lower ionic strength and higher temperature, such as 0.2 × SSC, 0.1%SDS, 60 °C; or (2) addition of denaturants during hybridization, such as 50% (v/v) formamide, 0.1% calf serum/0.1% Ficoll, 42 °C, etc; or (3) hybridization that occurs only when the identity between two sequences is at least more than 90%, or preferably, more than 95%. In some embodiments, the hybridizable polynucleotide encodes a polypeptide that has the same biological function and activity as the mature polypeptide set forth in SEQ ID NO: 1 or 2.

The sequence of the variant described in the description may be at least 95%, 96%, 97%, 98% or 99% identical to its source sequence. The sequence identity described in the description can be measured using sequence analysis software. For example, the computer program BLAST with default parameters, especially BLASTP or BLASTN.

The full-length sequence of the fusion protein coding sequence of the present description or its fragments (such as primers or probes) can usually be obtained by PCR amplification, recombination or artificial synthesis. For the PCR amplification method, primers can be designed based on the relevant amino acid or nucleotide sequences disclosed in the present description, especially the open reading frame sequence, and commercially available DNA libraries or cDNA libraries prepared according to conventional methods known to those skilled in the art are used as templates to amplify the relevant sequences. In addition, methods of artificial synthesis can also be used to synthesize relevant sequences, especially when the fragment is short, such as primers or probes. Methods known in the art for designing primer and probe sequences can be used herein, for example through the software Primer Express.

The present description also relates to nucleic acid constructs containing the polynucleotide sequences described herein, and one or more regulatory sequences operably linked to these sequences. The polynucleotides of the description can be manipulated in a variety of ways to ensure expression of the fusion protein. The nucleic acid construct can be manipulated according to the difference or requirements of the expression vector prior to insertion of the nucleic acid construct into the vector. Techniques that utilize recombinant DNA methods to alter polynucleotide sequences are known in the art.

Regulatory sequences may be suitable promoter sequences. The promoter sequence is usually operably linked to the coding sequence of the protein to be expressed. The promoter can be any nucleotide sequence that shows transcriptional activity in the selected host cell, including mutated, truncated, and hybrid promoters, and can be derived from genes encoding extracellular or intracellular polypeptides homologous or heterologous to the host cell. The regulatory sequence may also be a suitable transcription terminator sequence, a sequence recognized by a host cell to terminate transcription. A terminator sequence is operably linked to the 3' terminal of the nucleotide sequence encoding the polypeptide. Any terminator that is functional in the selected host cell may be used in the present description. The regulatory sequence may also be a suitable leader sequence, an untranslated region of the mRNA important for translation in the host cell. A leader sequence is operably linked to the 5' terminal of the polypeptide-coding nucleotide sequence. Any terminator that is functional in the selected host cell may be used in the present description.

In certain embodiments, the nucleic acid construct is a vector. The vector can be a cloning vector, an expression vector, or an integration vector. The polynucleotides of the present description can be cloned into many types of vectors, e.g., plasmids, phagemids, phage derivatives, animal viruses, and cosmids. Cloning vectors can be used to provide coding sequences for the fusion proteins of the description. Expression vectors can be provided to cells in the form of viral vectors. Expression of the present polynucleotides is generally achieved by operably linking the polynucleotides of the description to a promoter and incorporating the construct into an expression vector. Integration vectors are used to integrate the expression cassette described herein into the host genome, such as integration vectors that integrate genes through the PB transposition system, such as pNB, pNC. Typically, a suitable vector will contain an origin of replication functional in at least one organism, a promoter sequence, transcriptional and translational terminators, convenient restriction enzyme sites, and/or one or more selectable markers. These sequences can be selected as needed by those skilled in the art.

To assess protein expression, expression vectors introduced into cells may also contain selectable marker genes and/or reporter genes to facilitate the identification and selection of expressing cells from the population of cells sought to be transfected or infected by the viral vector. Selectable markers can be carried in a single DNA fragment and used in co-transfection procedures. Both the selectable marker and the reporter gene may be flanked by appropriate regulatory sequences to enable expression in the host cell. Useful selectable markers include, for example, antibiotic resistance genes such as neo and the like. Suitable reporter genes may include genes encoding luciferase, β-galactosidase, chloramphenicol acetyltransferase, secreted alkaline phosphatase, or the green fluorescent protein gene. Suitable expression systems are well known and can be prepared using known techniques or obtained commercially.

The nucleic acid constructs described herein can generally be obtained by PCR amplification. Specifically, primers can be designed according to the nucleotide sequence disclosed herein, especially the open reading frame sequence, and a commercially available cDNA library or a cDNA library prepared by a conventional method known to those skilled in the art can be used as a template for and amplification of related sequences. When the sequence is long, it is often necessary to carry out two or more rounds of PCR amplifications, and then assemble the amplified fragments in correct order. Alternatively, the nucleic acid molecules described herein can also be directly synthesized.

Methods of introducing genes into cells and expressing genes in cells are known in the art. Vectors can be readily introduced into host cells, eg, mammalian, bacterial, yeast or insect cells, by any method known in the art. For example, expression vectors can be transfected into host cells by physical, chemical or biological means.

Physical methods of introducing polynucleotides into host cells include calcium phosphate precipitation, lipofection, particle bombardment, microinjection, electroporation, and the like. Biological methods for introducing polynucleotides of interest into host cells include the use of DNA and RNA vectors. Chemical means of introducing polynucleotides into host cells include colloidal dispersion systems, such as macromolecular complexes, nanocapsules, microspheres, beads; and lipid-based systems, including oil-in-water emulsions, micelles, mixed micelles, and liposome.

As used herein, a host cell contains, expresses and/or secretes a fusion protein described herein. In the present description, when it is mentioned that a cell contains or harbors, expresses, or secretes a certain molecule such as a polypeptide, "contain" means that the molecule is contained in or on the surface of the cell; "express" means that the cell produces the molecule; "secrete" means that the cell secretes the expressed molecule out of the cell. Host cells include immune cells (such as T cells) ultimately used for disease treatment purposes, and various types of cells used during the production of immune cells, such as E. *coli* cells, e.g., to provide the coding sequence of the protein or vectors described herein. Proteins expressed and/or secreted by cells can be purified by purification methods well known in the art.

T cells suitable for use in the present description can be various types of T cells from various sources. For example, T cells can be derived from PBMC of healthy individuals. In certain embodiments, the present description provides a TCR-T cell, CAR-T cell, or TIL that stably expresses the fusion protein described herein. These methods are known to those skilled in the art. In some embodiments, the obtained T cells can be first stimulated and activated with an appropriate amount (for example, 30-80ng/ml, such as 50ng/ml) of CD3 antibodies, and then cultured in a medium containing an appropriate amount of IL2 (for example, 30 to 80 IU/ml, such as 50 IU/ml) for later use.

Therefore, in certain embodiments, the present description provides a genetically modified cell (e.g., an immune cell) that contains a nucleic acid molecule as described herein, or contains a vector as described herein, or is prepared using the methods described herein, or stably expresses the fusion protein described herein.

The present description also provides a pharmaceutical composition, which contains any one or more of fusion proteins, nucleic acid molecules, nucleic acid constructs and cells, and pharmaceutically acceptable excipients. As used herein, pharmaceutically acceptable excipients refer to carriers and/or excipients that are pharmacologically and/or physiologically compatible with the subject and the active ingredient. In certain embodiments, acceptable excipients and the like in pharmaceutical compositions are preferably non-toxic to the recipient at the doses and concentrations employed. In certain embodiments, pharmaceutical compositions may contain ingredients for improving, maintaining or preserving, for example, the pH, osmolarity, viscosity, clarity, color, isotonicity, odor, sterility, stability, dissolution or release rate, absorption or penetration. These ingredients are known in the art and include, but are not limited to: pH adjusters, surfactants, ionic strength enhancers, diluents, vehicles, solubilizers, emulsifiers, preservatives and/or adjuvants. More specifically, suitable pharmaceutically acceptable excipients may be excipients commonly used in the art for the administration of immune cells, especially T cells. See, for example, REMINGTON'S PHARMACEUTICAL SCIENCES, 18th ed., edited by A. R. Genrmo, 1990, Mack Publishing Company. The optimal pharmaceutical composition can be determined according to the expected route of administration, mode of delivery, and required dose.

The pharmaceutical composition of the present description may be selected for parenteral delivery. Alternatively, compositions may be selected for inhalation or delivery through the digestive tract, such as oral delivery. The preparation of the pharmaceutically acceptable composition is within the art. Other pharmaceutical compositions will be obvious to those skilled in the art, including formulations comprising immune cells, particularly immune cells (eg, T cells), in sustained or controlled release delivery formulations. The techniques used to prepare a variety of other sustained or controllable delivery modes (such as liposome carriers, bioerodible particles or porous beads, and deposit injection) are also known to those skilled in the art. Cell-containing pharmaceutical compositions can be injected directly into tumors, lymph nodes, or sites of infection.

Pharmaceutical compositions for in vivo administration are usually provided in the form of sterile formulations. Sterilization is achieved by filtration through a sterile filter membrane. Compositions for parenteral administration may be stored in lyophilized form or in solution (e.g., cryopreserved preparations). Parenteral compositions are usually placed in containers with sterile access holes, such as intravenous solution strips or vials with plugs that can be pierced by subcutaneous injection needles.

Once the pharmaceutical composition is prepared, it is stored in sterile vials in the form of solution, suspension, gel, emulsion, solid, crystal, cryopreservant or dehydrated or lyophilized powder. The pharmaceutical formulations (e.g., cryopreserved formulations) can be stored in ready-to-use form or further formulated prior to administration. For example, pharmaceutical compositions described herein that are suitable for delivery may be cryopreserved formulations that can tolerate long-distance transport without damaging the cells. In addition to the cells themselves, cryopreservation preparations usually further include components such as cell cryopreservation solution and human serum albumin (HSA). Prior to administration (e.g., intravenous infusion), the cryopreserved pharmaceutical composition needs to be stored at low temperature (e.g., in liquid nitrogen). After thawing, the cryopreserved preparation can be directly or formulated into an infusion composition for infusion to the patient. Those skilled in the art are aware of the components and concentrations of conventional cryopreservation solutions. For example, the cryopreservation solution or infusion composition may also contain sodium chloride, glucose, sodium acetate, potassium chloride or magnesium chloride, etc., the concentration of which can be determined by those skilled in the art (for example, an experienced physician) according to the conditions of cells, diseases, patients, etc. The description also provides a kit for generating a single dose administration unit. The kits of the present description may each contain a first container harboring a cell cryopreservation solution or cryopreservation preparation and optionally a second container harboring an infusion formulation. In certain embodiments of the description, kits are provided containing single and multi-lumen prefilled infusion sets (e.g., liquid infusion sets).

Also provided herein is a kit containing a nucleic acid construct described herein. The kit may also contain various reagents suitable for transfecting the nucleic acid construct into cells, and optional instructions to guide those skilled in the art to transfect the recombinant expression vector into cells.

The description also provides methods of treating a disease in a patient by administering a fusion protein, cell or pharmaceutical composition according to any embodiment of the description. The diseases described in the present description include various types of cancer (tumor), including solid tumors (such as adenocarcinoma, lung cancer, colon cancer, large intestine cancer, breast cancer, ovarian cancer, cervical cancer, gastric cancer, bile duct cancer, gallbladder cancer, esophageal cancer, pancreatic cancer and prostate cancer) and blood tumors, as well as leukemias and lymphomas, such as B-cell lymphoma, mantle cell lymphoma, acute lymphoblastic leukemia, chronic lymphocytic leukemia, hairy cell leukemia and acute myeloid leukemia. Preferably, the disease is a tumor that benefits from dimerization or multimerization of a functional domain described herein (an antibody or cytokine described herein).

Terms "patient", "individual" and "subject" are used interchangeably herein, including any organism, preferably animals, more preferably mammals (such as rats, mice, dogs, cats, rabbits, etc.), and most preferably humans. "Treatment" refers to the subject accepting the treatment scheme described herein to achieve at least one positive therapeutic effect (for example, a reduced number of cancer cells, a reduced tumor volume, a reduced rate of cancer cells infiltrating into peripheral organs, or a reduced rate of tumor metastasis or tumor growth). Typically, a therapeutically effective amount cells is included in the pharmaceutical composition. A therapeutically effective amount refers to a dose that can achieve cure, prevention, alleviation and/or amelioration of a disease or condition in a subject. The therapeutically effective dose can be determined according to factors such as the patient's age, sex, disease and its severity, and other physical conditions of the patient. Herein, a subject or a patient generally refers to a mammal, especially a human.

The therapeutically effective amount of a pharmaceutical composition containing a fusion protein or immune cell of the description to be employed will depend, for example, on the process and goals of the treatment. Those skilled in the art will understand that the appropriate dose level for treatment will vary in part depending on the molecule delivered, the indication, the route of administration, and the size (body weight, body surface or organ size) and/or conditions (age, general health condition, and the potential of the therapy to stimulate an anti-cancer response in the subject) of the patient. In some embodiments, clinicians may determine the dose through titration and change the route of administration to obtain the optimal therapeutic effect.

The frequency of dosing will depend on the pharmacokinetic parameters of the immune cells in the formulation used. Clinicians typically administer the compositions until reaching a dosage that achieves the desired effect. The composition may therefore be administered as a single dose, or as two or more doses over time (which may or may not contain the same amount of the desired molecule), or by a continuous infusion through an implanted device or catheter.

The route of administration of the pharmaceutical composition is generally a known method, such as oral, intravenous, intraperitoneal, intracerebral (intraparenchymal), intraventricular, intramuscular, intraocular, intra-arterial, portal vein or intralesional injection; by continuous release system or by implantable device.

In some embodiments of the description, the fusion proteins, cells or pharmaceutical compositions of the description can be combined with other therapies known in the art. Such therapies include, but are not limited to, chemotherapy, radiotherapy, and immunosuppressants. For example, treatment may be combined with radiotherapy or chemotherapy agents known in the art to treat tumor antigen-mediated diseases.

The present description also provides the use of any one or more of the fusion proteins, nucleic acid molecules, nucleic acid constructs, cells and pharmaceutical compositions described in any embodiment herein in the preparation of anti-tumor drugs. Herein, "anti-tumor" refers to a biological effect, which can be manifested by a decrease in tumor volume, in the number of tumor cells, in the number of metastases, an increase in life expectancy, or an improvement in various physiological symptoms related to cancer.

The present description is described in further detail with reference to the following experimental examples. These examples are provided for illustrative purposes only and are not intended to be limiting unless otherwise specified. Accordingly, the present description should in no way be construed as limited to the following examples, but should be construed to include any and all changes that become apparent in light of the teachings provided herein. Unless otherwise specified, the methods and materials used in the examples are conventional materials and methods in the art.

### EXAMPLE

### Antibodies and Instruments

OKT3 purchased from: ThermoFisher, Cat. No.: 14-0037-82
CD28 antibody was purchased from: ThermoFisher Cat. No.: 14-0281-82
Electroporation instrument: Lonza Nucleofector 2b purchased from Lonza Cat. No.: AAB-1001

### Example 1: Construction of expression vector for CD52 linker-anchored membrane-bound protein

The coding sequences containing the following CD52 linker-anchored membrane surface proteins were synthesized:
1. Amino acid sequences and coding sequences of the fusion proteins of IL-7 and CD52 reading frames:
   IL-7-CD52-1: CD52 signal peptide-IL-7-CD8 extracellular domain-CD52, coding sequence: SEQ ID NO: 1; protein sequence: SEQ ID NO: 2;
   IL-7-CD52-2: CD52 signal peptide-IL-7-linker-BCMA extracellular region -CD52, coding sequence: SEQ ID NO: 3; protein sequence: SEQ ID NO: 4;
   IL-7-CD8TM: CD8 signal peptide-IL-7-CD8 extracellular region-CD8 transmembrane region, coding sequence: SEQ ID NO: 5; protein sequence: SEQ ID NO: 6;
2. Amino acid sequences and coding sequences of the fusion proteins of HER2 antibody and CD52 reading frame:
   αHer2-CD52: CD52 signal peptide-αHer2-linker-CD52, coding sequence: SEQ ID NO: 7, protein sequence SEQ ID NO: 8;
3. Amino acid sequences and coding sequences of the fusion proteins of CD137 activating antibody and CD52 reading frame:
   αCD137-CD52: CD52 signal peptide-αCD137-linker-CD52 sequence: coding sequence: SEQ ID NO: 9; protein sequence SEQ ID NO: 10;

The pKB20 vector was constructed according to the method described in Example 1 on page 21 of the PCT application WO2022078310A1 specification. According to the method described in this example, pKB20 vectors containing the expression cassettes of IL-7-CD52-1, IL-7-CD52-2, IL-7-CD8TM, αHer2-GPI and αCD137-GPI were constructed and named pKB20-IL-7-CD52-1, pKB20-IL-7-CD52-2, pKB20-IL-7-CD8TM, pKB20-αHer2-CD52 and pKB20-αCD137-CD52, respectively. According to the method described in the same example, the EGFP-expressing vector pKB20-EGFP was constructed. The obtained recombinant plasmid was transformed into E. coli (DH5c). After confirming the correctness of the sequence through sequencing, the plasmid was extracted and purified using Qiagen's plasmid purification kit to obtain high-quality plasmids of each recombinant expression vector.

### Example 2: Preparation of tumor antigen NY -ESO-1 TCR expression vector

The DNA sequences encoding the genes of α chain and β chain of the TCR that recognizes the NY-ESO-1 antigenic peptide SLLMWITQC (HLA-*A02:01) were synthesized, and the two chains were linked by the DNA sequence encoding the P2A peptide. The assembled sequence is as shown in SEQ ID NO: 11. Then, the DNA sequence encoding EGFP was connected to the 3' end of SEQ ID NO: 11 through the DNA encoding the P2A peptide, and the resulting sequence is as shown in SEQ ID NO: 12. SEQ ID NO: 12 was commercially synthesized and cloned into a prepared pKB20 vector according to the method described in Example 1 on page 21 of the specification of WO2022078310A1, and the obtained vector is named pKB20-TCR. The vector pKC20, which does not contain the PB transposase expression cassette, was prepared according to the method described in Example 1 on page 21 of the specification of WO2022078310A1. According to the method described in the same example, SEQ ID NO: 12 was cloned into the prepared pKC20 vector and the obtained vector is named pKC20-TCR. The obtained recombinant plasmid was transformed into E. coli (DH5c). After confirming the correctness of the sequence through sequencing, the plasmid was extracted and purified using Qiagen's plasmid purification kit to obtain high-quality plasmids for each recombinant expression vector.

### Example 3: Preparation and culture of T cells expressing CD52 linker-anchored membrane-bound IL-7

According to the following steps, PBMCs were electroporated using the expression vector in Example 1 to prepare T cells expressing CD52 linker-anchored membrane-bound IL-7. The PBMCs used were purchased from AllCells Company and came from the peripheral blood of healthy adults.
1) Collect the suspended cells in a 50ml centrifuge tube, centrifuge at 1200rmp for 3min;
2) Discard the supernatant, resuspend cells in normal saline, centrifuge at 1200rmp for 3min, discard the normal saline, and repeat this step, then perform cell counting;
3) Add 5×10⁶ cells into five 1.5ml centrifuge tubes each, and centrifuge at 1200rmp for 3min;
4) Discard the supernatant, prepare the electroporation kit (purchased from Lonza) for use, add 18 µL of solution I reagent and 82 µL of solution II reagent, add 5µg pKB20-IL-7-CD52-1 plasmid to Tube 1, add 5µg pKB20-IL-7-CD52-2 plasmid to Tube 2, add 5µg pKB20-IL-7-CD8TM plasmid to Tube 3, and add 5 µg of pKB20-EGFP plasmid to each of Tubes 4 and 5;
5) Transfer the cell suspension mixed with the plasmid in the centrifuge tube to the electroporation cuvette, put it into the electroporator, select program T020, and apply electroporation;
6) Transfer the electroporated cell suspension to a 6-well plate pre-filled with AIM-V culture medium containing 2% FBS using the micropipette in the kit, mix well, and place it in a 37°C, 5% CO₂ incubator for 4-6 hours; meanwhile, coat 5 wells of a 6-well plate with a mixture containing 5 µg/mL OKT-3 and 5 µg/mL CD28 antibodies, add 1 mL of the mixture to each well, and incubate the 6-well plate at 37°C;
7) After 6 hours, remove the antibody coating mixture and wash twice with PBS. Cells cultured in a 37°C, 5% CO₂ incubator after electroporation were transferred into the 6-well plate pre-coated with OKT-3 and CD28 antibodies. To each well 3 mL of culture medium was added, and IL-2 was not added to the cell culture medium for pKB20-IL-7-CD52-1, pKB20-IL-7-CD52-2, and pKB20-IL-7-CD8TM. IL-2 was not added to the culture medium in one of the two wells of cells electroporated with pKB20-EGFP (pKB20-EGFP-IL-2-), and for the other well, IL-2 was added to the culture medium to a final concentration of 500U/mL (pKB20-EGFP-IL-2+). The growth status of cells in each group was monitored.

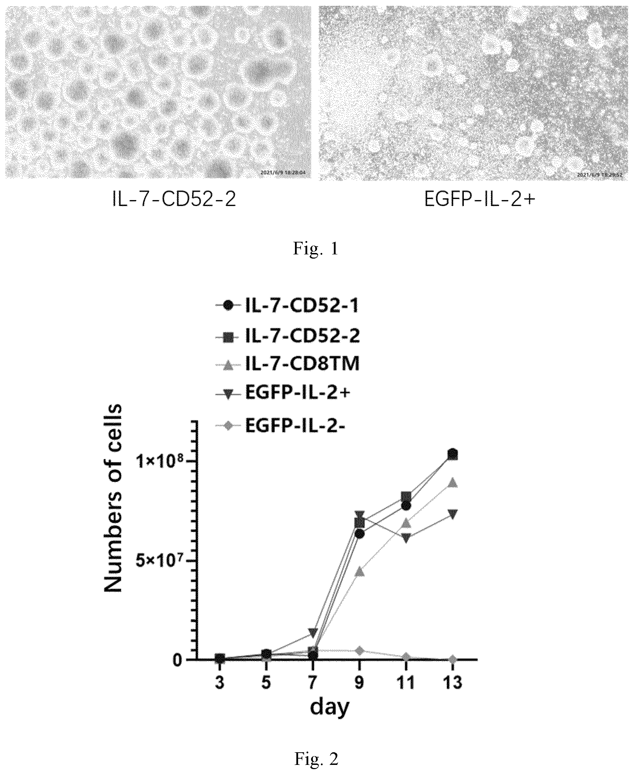

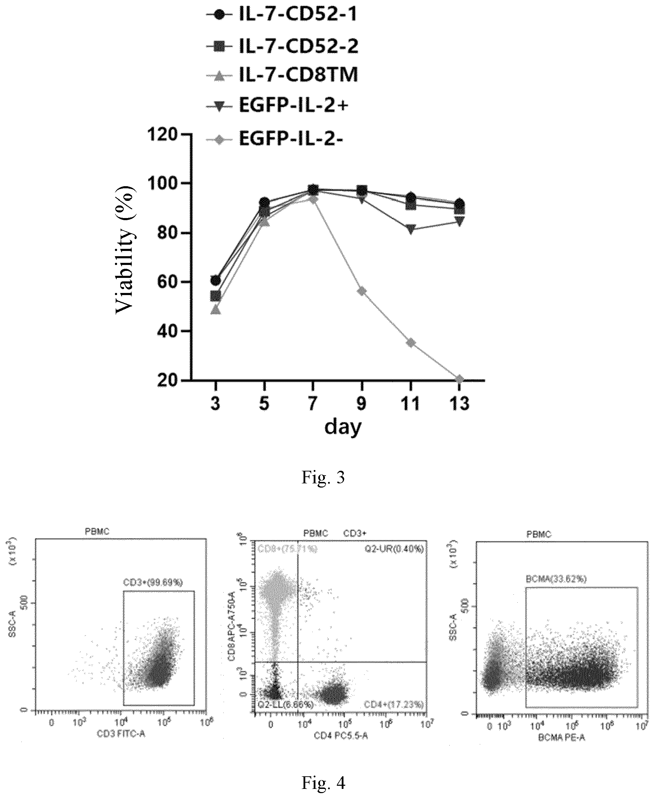

The results were shown in Fig 1, Fig 2 and Fig 3. Figure 1 shows microscopic morphology of T cells electroporated with pKB20-IL-7-CD52-2 ( IL-7-CD52-2 ) and T cells electroporated with pKB20-EGFP and cultured in the presence of IL-2 (EGFP-IL-2+ ). Compared with EGFP-IL-2+ T cells, the aggregation of IL-7-CD52-2 T cells was more pronounced, while the aggregation of T cells during growth is usually related to increase in self-activation. Figure 2 shows that after 13 days of culture post-electroporation, the number of pKB20-IL-7-CD52-1 cells and pKB20-IL-7-CD52-2 cells was remarkably higher than that of pKB20-IL-7-CD8TM cells, and the number of cells in the above three groups was significantly higher than that of pKB20-EGFP-IL-2+ cells. No obvious cell proliferation was found in pKB20-EGFP-IL-2- cells. Figure 3 shows that pKB20-IL-7-CD52-1, pKB20-IL-7-CD52-2 and pKB20-IL-7-CD8TM cells have similar cellular viability, all above 90%, and slightly higher than that of pKB20 -EGFP-IL-2+ cells. The cellular viability of pKB20-EGFP-IL-2- cells began to decline 7 days after culture and had fallen to about 20% by 13 days after electroporation. The results in Figure 1 to Figure 3 show that IL-7 anchored on the membrane surface can replace IL-2 in the cell culture medium and generate significant activation and proliferation effects on PBMCs, and the stimulation and proliferation effects on PBMC produced by IL-7 anchored on the cell membrane surface via CD52 GPI are better than those produced by IL-7 anchored on the cell membrane surface via conventional polypeptide transmembrane domain.

### Example 4: Detection of the proportion of T cells expressing CD52 linker-anchored membrane-bound IL-7

A. Indirect method to detect the proportion of cells expressing cell membrane- bound IL-7. The method is as follows:
   1) Collect cells from groups pKB20-IL-7-CD52-1, pKB20-IL-7-CD52-2 and pKB20-IL-7-CD8TM, respectively, 1×10⁶ cells each, and centrifuge at 1000rpm for 3 minutes;
   2) Discard the supernatant, add normal saline to re-suspend the cells, and centrifuge at 1000rpm for 3min;
   3) Discard the supernatant, and add 100 µL of normal saline to each tube to re-suspend the cells. Add 1 µL of biotin-labeled anti-IL-7 antibody (purchased from Biolegend, Cat. No.: 506602) to each tube, and incubate at 4°C for 30 minutes;
   4) Add appropriate amount of normal saline to each tube, centrifuge at 1000rpm for 3min, wash twice, and discard the supernatant;
   5) Add 100 µL of normal saline to each tube to re-suspend the cells. Add 1 µL of PE-labeled streptavidin (purchased from Thermo Fisher, Cat NO: S20982) to each tube, mix well, and incubate at 4°C for 30 min;
   6) Add an appropriate amount of normal saline to each tube, centrifuge at 1000rpm for 3min, wash twice, and discard the supernatant;
   7) Re-suspend cells with 400 µL of normal saline, and submit to flow cytometric analysis.
      Flow cytometric results show that the proportions of cells positive for exogenous gene expression in the groups pKB20-IL-7-CD52-1, pKB20-IL-7-CD52-2 and pKB20-IL-7-CD8TM were all above 30%.
B. Use the BCMA extracellular domain in the extracellular region of cells from the group pKB20-IL-7-CD52-2 as label. Use the cells from group pKB20-IL-7-CD52-2 obtained in Example 3 to measure the proportion of cells positive for exogenous gene expression using the direct method via flow cytometry. The method is as follows:
   1) Collect 1×10⁶ cells from group pKB20-IL-7-CD52-2 and centrifuge at 1000rpm for 3min;
   2) Discard the supernatant, and add normal saline to re-suspend the cells. Then centrifuge at 1000rpm for 3min;
   3) Discard the supernatant, and add 100 µL of normal saline to each tube to re-suspend the cells. Add 2 µL of BCMA flow cytometry antibody to each tube (purchased from Biolegend, Cat. No.: 357504), and incubate at room temperature for 30 minutes;
   4) Add an appropriate amount of normal saline, centrifuge cells at 1000rpm for 3min, wash twice, and discard the supernatant;
   5) Re-suspend cells with 400 µL of normal saline, and submit to flow cytometry.

The results were shown in Figure 4. The proportion of BCMA+ cells in Group pKB20-IL-7-CD52-2 was 33.62%, indicating that the proportion of positive cells in Group pKB20-IL-7-CD52-2 was over 30%, which was basically consistent with the results obtained via indirect method.

### Example 5: Detection of IFN-γ secretion by T cells expressing CD52 linker-anchored membrane-bound IL-7

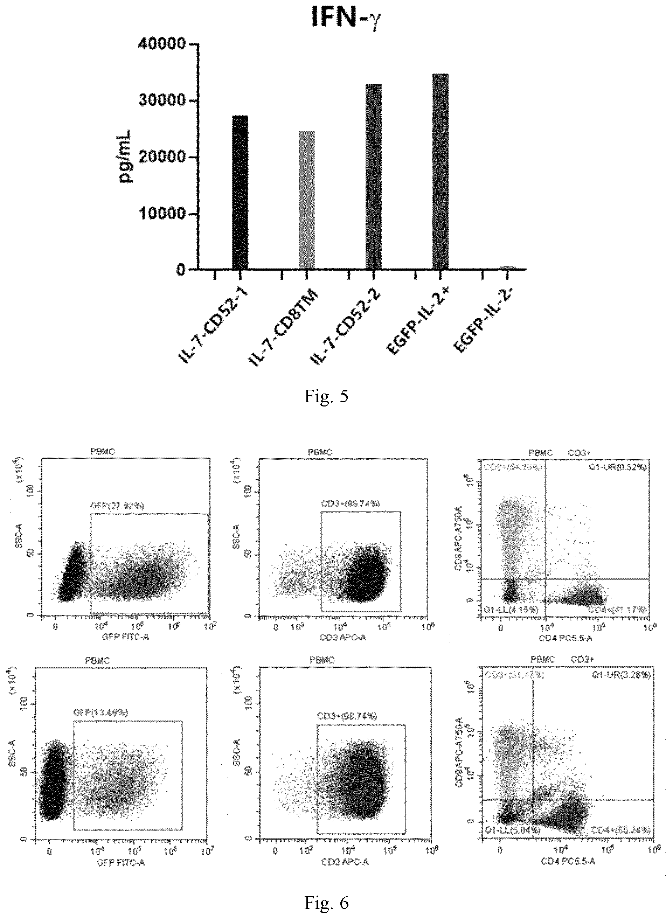

Harvest cells from each group prepared in Example 3, and use the HTRF IFN-γ detection kit (Cisbio Human IFN gamma kit, Cat. NO.: 62HIFNGPET) to measure the IFN-γ secretion level according to the method described in the user manual. The results were shown in Figure 5. Figure 5 shows that the concentration of IFN-γ secreted into the supernatant by cells in each group exceeded 25000 pg/mL. Among them, the IFN-γ secretion level of cells from the group pKB20-IL-7-CD52-2 is higher than that of cells from groups pKB20-IL-7-CD52-1 and pKB20-IL-7-CD8TM, comparable to the secretion level of cells from group pKB20-EGFP-IL-2+. This indicates that CD52-anchored expression on the surface of T cells achieves a similar cytokine secretion effect as incubation with IL-2 does.

### Example 6: Preparation of TCR-T expressing CD52 linker-anchored membrane-bound IL-7

### Preparation of activated human T cells:

Coat six-well plates with a coating solution containing 5 µg/ml anti-CD3 antibody and 5 µg/ml anti-CD28 antibody at room temperature for 2-4 hours. After removing the coating solution, wash the plates with normal saline for 1-3 times, and add AIM-V medium containing 2% FBS. Recover human PBMC (HLA-*02:01, purchased from ALLCELLS) in a 37°C water bath. Culture the PBMC adherently for 2-4 hours, and the suspension cells that do not attach are the initial T cells. Add the suspension cells into a 15ml centrifuge tube, centrifuge at 1200rmp for 3min, and discard the supernatant. Add normal saline, centrifuge at 1200rmp for 3min, and then discard the normal saline. Repeat this step, then transfer the washed naive T cells to the antibody-coated wells filled with ready-to-use culture medium, culture at 37°C, 5% CO₂ for 3 to 4 days before subsequent experiments.

Prepare TCR-T cells expressing NY-ESO-1 TCR-EGFP and TCR-T cells expressing NY-ESO-1 TCR-EGFP+CD52 linker-anchored membrane-bound IL-7 by electroporation:
1) Add AIM-V medium to 2 of the wells in a 12-well plate in advance, 2 mL per well, and then transfer the plate to a cell culture incubator and sit for one-hour at 37°C, 5% CO₂ for preheating;
2) Prepare the electroporation solution for each well in a single experiment according to the following table for 2 wells:

**Table 1**

| | 100 µL Nucleocuvette^{™} Strip (µL) |
|---|---|
| Volume of Nucleofector^{™} solution | 82 |
| Electroporation Supplement | 18 |

3) Transfer the obtained activated T cells into two EP tubes, 5×10⁶ cells for each EP tube, and centrifuge at 1200 rpm for 5 min. Discard the supernatant, and re-suspend the cells with 500 µL of normal saline. Repeat centrifuge to wash the cell pellet;
4) Add 6 µg of the plasmid pKB20-TCR to one of the two wells containing the electroporation mixture prepared in step 2). Add 3 µg of pKB20-IL-7-CD52-2 and 3 µg of pKB20-IL-7-CD52-2 to the other well, and sit at room temperature for 30 minutes;
5) Re-suspend the two tubes of activated T cells with the electroporation solution containing the plasmids prepared in 4), 100 µL in each tube. Carefully pipette the cell re-suspension into a LONZA 100 µL electroporation cuvette, and then place the cuvette into the LONZA Nucleofector^{™} 2b electroporation chamber. Select electroporation program T-020 for execution;
6) After completion of the electroporation, remove the electroporation cuvettes with care. Transfer the cell suspension to EP tubes which have been prefilled with 200 µL of preheated AIM-V medium each, and then transfer the cells into the wells containing preheated AIM-V culture medium in the 12-well plate prepared in step 1) and culture at 37°C and 5%CO₂. After culturing the cells for 1 h, add the compound G150 (purchased from MedChemExpress) to a final concentration of 5 µM, and then culture the cells for 13 days, during which the cells are passaged according to their proliferation status. After 13 days, submit each electroporated sample to total cell counting and cell viability test.

The cells expressing exogenous genes prepared by the above method were named TCR-T and IL-7-CD52-TCR-T, respectively.

Submit the obtained cells to flow cytometric analysis (Beckman Cytoflex) to determine the proportion of EGFP-positive cells. For TCR-T, EGFP-positive cells can be viewed as cells positive for NY-ESO-1 TCR gene expression. For cells co-transfected with IL-7-CD52-2 and TCR, EGFP positivity indicates that the PB transposase which is essential for the successful integrated expression of TCR-EGFP comes from the pKB20 vector that expressed the IL-7-CD52-2 encoding gene, as the pKC20 vector does not contain the PB transposase expression cassette. Therefore, for cells co-transfected with IL-7-CD52-2 and TCR, EGFP-positive cells represent dual integrated expression of IL-7-CD52-2 and NY-ESO-1 TCR.

The obtained flow cytometric results were shown in Figure 6. The first line in Figure 6 is the result of TCR-T flow cytometric assay. The second line is the result of IL-7-CD52-TCR-T. Figure 5 shows that the proportion of TCR integrated expression-positive cells in TCR-T was 27.92%, and the proportion of cells positive for dual integration of IL-7-CD52-2 and TCR in IL-7-CD52-TCR-T was 13.48% with GFP-positive cells as an indicator.

### Example 7: Killing of target cells by TCR-T expressing CD52 linker-anchored membrane-bound IL-7

HLA-A*02: 01 melanoma cell line A375 positive for NY-ESO-1 (purchased from American Type Culture Collection, ATCC, Cat. CRL-1619 ) was used as the target cell, and the Agilent real-time label-free cell function analyzer (RTCA) was used to measure the *in vitro* killing activity of the TCR-T and IL-7-CD52-TCR-T cells prepared in Example 6. Specific steps are as follows:
(1) Zero adjustment: add 50 µL of DMEM or 1640 culture solution to each well, put the well into the instrument. Start zero adjustment by selecting step 1;
(2) Plating of target cells: plate melanoma cells A375 at the density of 10⁴ cells/50 µL per well on a plate equipped with detection electrodes, sit for a few minutes until the cells settle down, then place the well into the instrument. Start the cell culture by selecting step 2;
(3) Addition of effector cells: After culturing the target cells for 24 hours and the Cell Index reaches 1.0, pause step 2 and add 50 µL of effector cells into each well. Add effector cells to an effector-to-target ratio of 1:1 and 0.5:1, respectively according to the proportion of positive cells according to Example 6, then start step 3. Continue with the cell co-culture for over 80 hours, and observe the cell proliferation curve.

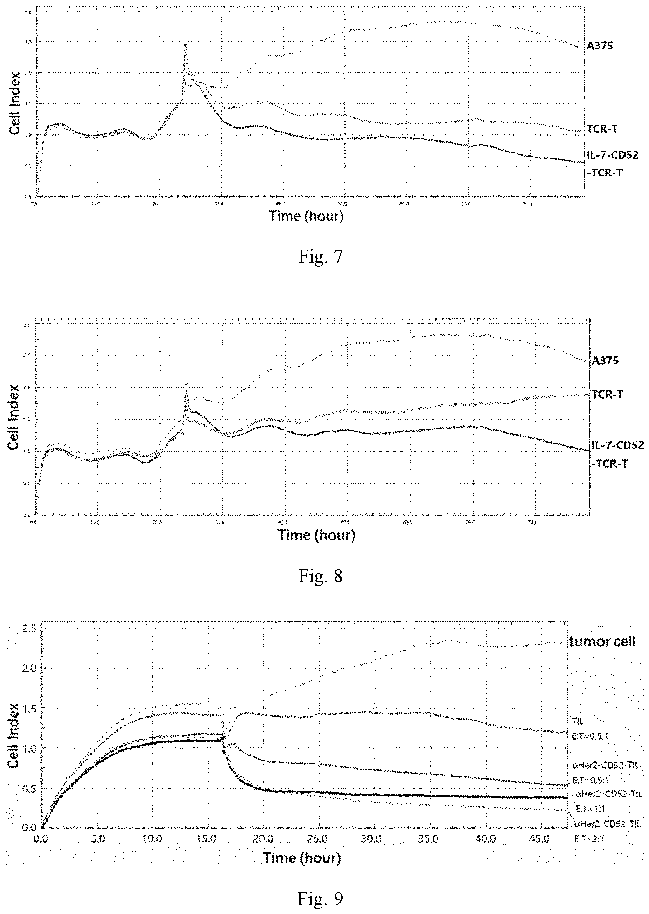

The results were shown in Figure 7 and Figure 8. Figure 7 and 8 show that the killing effect of IL-7-CD52-TCR-T on target cells A375 was significantly higher than that of TCR-T at the effector-to-target ratio of 1:1 and 0.5:1, indicating that the expression of CD52 linker-anchored IL-7 can significantly improve the recognition of antigens by TCR-T cells and the capacity of killing target cells.

### Example 8: Preparation and in vitro killing assay of tumor-infiltrating lymphocyte cells expressing CD52 linker-anchored membrane-bound anti-HER2 antibodies

### Preparation of tumor-infiltrating lymphocytes (TIL) from breast tumor tissue:

Freshly resected HER2+ breast cancer sample were collected and processed immediately under sterile conditions. The processing method is as follows: remove the normal tissue and necrotic areas around the breast tumor sample. Take small tissue fragments sized 1-2 mm³ from different areas of the sample, and place them into a 24-well plate, one per well. Add 2 mL of complete medium (AIM-V medium containing 10% FBS) and 3000 IU/mL IL-2 to each well. Place the 24-well plate at 37°C, 5% CO₂ in an incubator. From the 5th to 6th day after the initiation of the culture, replace half of the medium for all wells. Then replace half of the medium every 1-2 days according to the growth of TILs. Once TILs in the wells reach confluency and all the adherent cells are absent, harvest TILs that fill each well.

### Preparation of TILs expressing CD52 linker-anchored membrane-bound anti-HER2 antibodies and in vitro killing of primary tumor cells:

Electroporate the breast tumor TILs prepared above with the pKB20-αHer2-CD52 vector prepared in Example 1 according to the method described in Example 3 to obtain αHer2-CD52-TIL. Submit part of the same breast tumor tissue to enzymatic digestion to obtain single cell suspension. Plate the cell suspension in the wells of the RTCA analyzer according to the method described in Example 7. Adjust the time of adding effector cells to 16 hours after plating the target cells according to the growth rate of tumor cells. Establish groups TIL and αHer2-CD52-TIL. Set the effector-to-target ratio of group TIL to 0.5:1, set three effector-to-target ratios, 0.5:1, 1:1, and 2:1 for group αHer2-CD52-TIL, and observe the differences in the killing effects among different groups.

The results were shown in Figure 9. The killing effect of αHer2-CD52-TILs on primary breast cancer cells was significantly stronger than that of unmodified TILs at the same effector-to-target ratio (0.5:1). And as the effector-to-target ratio increases, the killing effect of αHer2-CD52-TILs on primary breast cancer cells also increases significantly, indicating that the expression of αHer2-CD52 on the surface of TIL can significantly enhance the killing effect of TIL on its corresponding patient-matched target cells.

### Example 9: Preparation of TCR-T expressing CD52 linker-anchored membrane-bound anti-CD137 antibody and in vitro killing of target cells

Co-transfect vectors pKB20-αCD137-CD52 and pKC20-TCR prepared in Example 1 into T cells according to the method described in Example 6 to obtain αCD137-CD52-TCR-T. The T cells used are from the same batch as those used in Example 6. Submit αCD137-CD52-TCR-T and the TCR-T prepared in Example 6 to RTCA killing assay on the target cells A375 according to the method described in Example 7. Adjust the time to add effector cells to 16 hours after plating the target cells in the wells according to the growth rate of A375 in the wells. Establish groups TCR-T and αCD137-CD52-TCR-T. Set the effector-to-target ratio of group TIL to 0.5:1, set three effect-to-target ratios, 0.5:1, 1:1, and 2:1 for group αCD137-CD52-TCR-T, and observe the differences in killing effects among different groups.

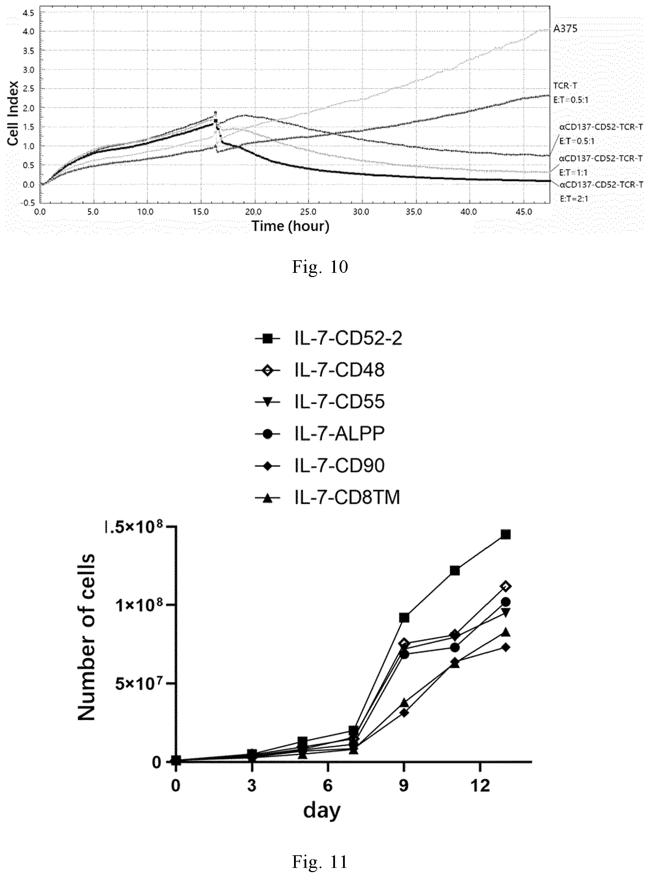

The results were shown in Figure 10, wherein the killing effect of αCD137-CD52-TCR-T on target cells A375 was significantly higher than that of unmodified TCR-T at under the same effector-to-target ratio (0.5:1). And as the effector-to-target ratio increases, the killing effect of αCD137-CD52-TCR-T on A375 cells also increases significantly, indicating that the expression of αCD137-CD52 on the surface of TCR-T can significantly enhance the killing effect of TCR-T on target cells.

### Example 10: Construction of expression vectors for membrane-bound IL-7 anchored to other GPI proteins

Construct the following IL-7-GPI anchor fusion proteins, respectively.
IL-7-CD48: CD48 signal peptide-IL-7-CD8 extracellular region-CD48 GPI signal sequence, its coding sequence: SEQ ID NO: 13; protein sequence: SEQ ID NO: 14;
IL-7-CD55: CD55 signal peptide-IL-7-CD8 extracellular region-CD55 GPI signal sequence, its coding sequence: SEQ ID NO: 15; protein sequence: SEQ ID NO: 16;
IL-7-ALPP: ALPP signal peptide-IL-7-CD8 extracellular region-ALPP GPI signal sequence, its coding sequence: SEQ ID NO: 17; protein sequence: SEQ ID NO: 18;
IL-7-CD90: CD90 signal peptide-IL-7-CD8 extracellular region-CD90 GPI signal sequence, its coding sequence: SEQ ID NO: 19; protein sequence: SEQ ID NO: 20;

The amino acid sequences of the CD48 GPI signal sequence, CD55 GPI signal sequence, ALPP GPI signal sequence and CD90 GPI signal sequence were shown in SEQ ID NO: 15-18 as described on pages 14 of the specification of CN110709416A, respectively.

Construct pKB20 vectors containing IL-7-CD 48, IL-7-CD 55, IL-7-ALPP and IL-7-CD90 expression cassettes named pKB20-IL-7-CD48, pKB20-IL-7-CD55, pKB20-IL-7-ALPP and pKB20-IL-7-CD90 according to the method described in Example 1, respectively. Transform the obtained recombinant plasmids into E. coli (DH5c). After confirming the correctness of the sequences through sequencing, extract and purify the plasmids using Qiagen's plasmid purification kit to obtain high-quality plasmid of each recombinant expression vector.

### Example 11: Preparation and culture of T cells expressing other GPI protein-anchored membrane-bound IL-7

Electroporate PBMC from healthy human donors with pKB20-IL-7-CD48, pKB20-IL-7-CD55, pKB20-IL-7-ALPP, and pKB20-IL-7-CD90 according to the method described in Example 3. Do not add IL-2 to the cell culture medium after electroporation, and prepare T cells expressing IL-7-CD48, IL-7-CD55, IL-7-ALPP and IL-7-CD90. Monitor the growth status of cells in each group, respectively, and compare with the T cells expressing IL-7-CD52 prepared in Example 3. Use T cells expressing IL-7-CD8TM as the control.

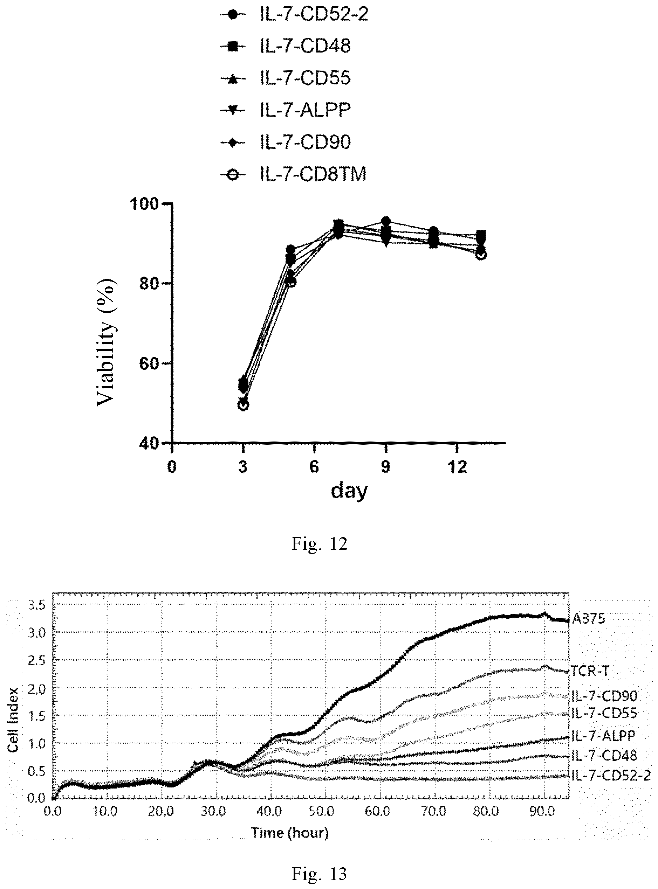

The results were shown in Figure 11 and Figure 12. Figure 11 shows that the proliferation level of T cells expressing IL-7-CD90 was basically equivalent to that of the control group expressing IL-7-CD8TM. The proliferation level of T cells expressing IL-7-CD48, IL-7-CD55, and IL-7-ALPP were significantly higher than that in the IL-7-CD8TM control group. The level of proliferation of T cells expressing IL-7-CD52-2 was higher than that of all other groups. Figure 12 shows that the viability of T cells electroporated with different membrane-bound IL-7 vectors of each group were basically identical at different time points.

The above results show that T cells expressing GPI protein-anchored membrane-bound IL-7 can increase the activation and proliferation level of T cells to varying degrees, wherein the activation and proliferation of T cells expressing CD52-anchored membrane-bound IL-7 is significantly stronger than that of T cells expressing membrane-bound IL-7 anchored by CD48, CD55, ALPP, and CD90.

### Example 12: Preparation and in vitro killing assay of TCR-T expressing other GPI protein-anchored membrane-bound IL-7

Co-transfect pKC20-TCR+pKB20-IL-7-CD48, pKC20-TCR+pKB20-IL-7-CD55, pKC20-TCR+pKB20-IL-7-ALPP and pKC20-TCR+pKB20-IL-7-CD90, respectively according to the method described in Example 6 to obtain IL-7-CD48-TCR-T, IL-7-CD55-TCR-T, IL-7-ALPP-TCR-T and IL-7-CD90-TCR-T. The proportions of positive cells for IL-7-CD48-TCR-T, IL-7-CD55-TCR-T, IL-7-ALPP-TCR-T and IL-7-CD90-TCR-T by flow cytometry were 15.09%, 16.74%, 12.53% and 17.66%, respectively.

Select A375 as target cells, and IL-7-CD48-TCR-T, IL-7-CD55-TCR-T, IL-7-ALPP-TCR-T, IL-7-CD90-TCR-T as well as TCR-T and IL-7-CD52-TCR-T prepared in Example 6 as effector cells according to the method described in Example 7. Perform the killing assay at the effector-to-target ratio of 0.5:1 based on the counting of living cells. Co-culture effector and target cells for over 90 hours, and observe the cell proliferation curve.

The results were shown in Figure 13. The killing effect of TCR-T expressing different GPI protein-anchored membrane-bound IL-7 on the target cell A375 in each group at the effector-to-target ratio of 0.5:1 was significantly better than that of TCR-T not expressing membrane-bound IL-7. Among TCR-T groups expressing GPI-anchored membrane-bound IL-7, IL-7-CD52-TCR-T expressing IL-7-CD52-2 exhibited a significantly better killing effect on target cells than IL-7-CD48-TCR-T, IL-7-CD55-TCR-T, IL-7-ALPP-TCR-T and IL-7-CD90-TCR-T did, indicating that the expression of GPI-anchored membrane-bound IL-7 can more effectively enhance the killing effect of TCR-T on target cells, while the expression of CD52- anchored membrane-bound IL-7 has a more pronounced effect on the killing effect of TCR-T on target cells compared with other GPI anchor proteins.

### Example 13: Killing effect of TCR-T expressing CD52 linker-anchored membrane-bound IL-7 on tumor tissue in vivo

### Experimental animals

Immunodeficient B-NDG mice (purchased from Biocytogen) were used as experimental animals to construct CDX models .

Experimental design and grouping are shown in Table 2 below

**Table 2 Dosing regimen and grouping of mice**

| Group | medicament | Animal numbers | Administration route | Frequency of administration | Administration volume (µL) | Time (days) |
|---|---|---|---|---|---|---|
| 1 | PBS | 6 | tail vein | Single | 200 | 20-25 |
| 2 | TCR-T (2×10⁷ /animal) | 6 | tail vein | Single | 200 | 20-25 |
| 3 | IL-7-CD52-2 TCR-T (2×10⁷ /animal) | 6 | tail vein | Single | 200 | 20-25 |

Administer TCR-T and IL-7-CD52-TCR-T prepared in Example 6 to the experimental animals. Centrifuge cells and re-suspend in PBS to obtain a PBS cell suspension with a cell density of 1×10⁸/mL before tail vein injection.

### Animal husbandry

Purchase the required number of B-NDG mice and keep in an SPF-level experimental animal room for 7-10 days of acclimatization.

Environment: mice will be housed in transparent resin plastic cages in the animal room. Cage litter is autoclaved wood chips and corncob litter replaced periodically. Animal rooms are equipped with high-efficiency air filters and will maintain at a temperature between 20-26°C (68-79°F) with a relative humidity of 40-70%. Temperature and humidity are observed and recorded continuously. The lighting conditions were 12 hours of daylight lamp illumination and 12 hours of darkness per day.

Food and drinking water: Experimental mice are allowed access to special mouse food (sterilized by irradiation, Shanghai SLAC Laboratory Animal, China) and sterilized drinking water ***ad libitum.***

### Tumor cell inoculation

Inject A375-Luc2 tumor cells (purchased from American ATCC, Cat. CRL-1619-LUC2) subcutaneously in each mouse at right side (2×10⁶ cells per mouse) in 0.1 mL of medium containing 50% matrix to promote tumor growth.

### Animal grouping and administration

When the tumor volume reaches ~50 mm³, randomly divide 18 animals into multiple groups according to the tumor volume with n=6, ensuring that all groups are comparable on the baseline. Record the day of grouping as P0. Measure the body weight and tumor volume of the animals twice a week during the experiment, and observe the clinical symptoms of the animals daily. The tumor volume is measured in mm³ according to the formula: V=0.5×a×b², wherein a and b are the long and short diameters of the tumor, respectively. Draw peripheral blood from mice in each group on the last day of the experiment, stain with human CD45 flow cytometric antibody (purchased from Biolegend Cat. 304036), and then subject the cells to flow cytometric assay. Inject luciferase substrate into mice in each group on the same day, allow reaction with luciferin, and perform ***in vivo*** fluorescence imaging on a small animal *in vivo* imaging system. Measure the size of tumor burden of the mice in each group and record the fluorescence values.

### Result

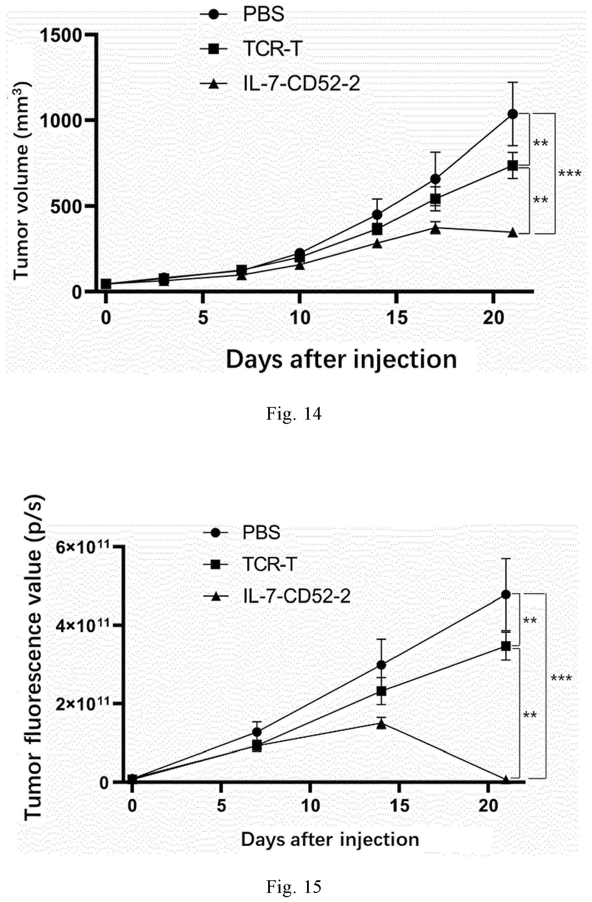

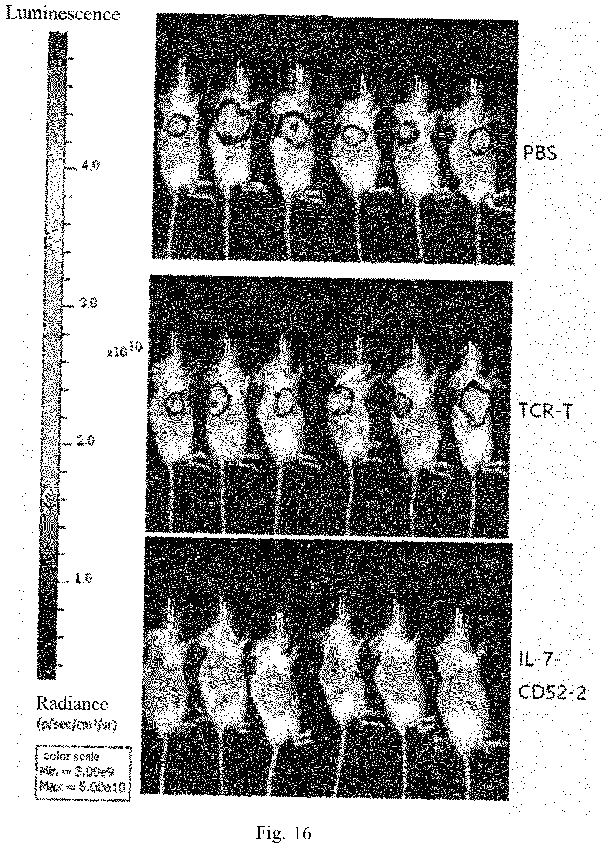

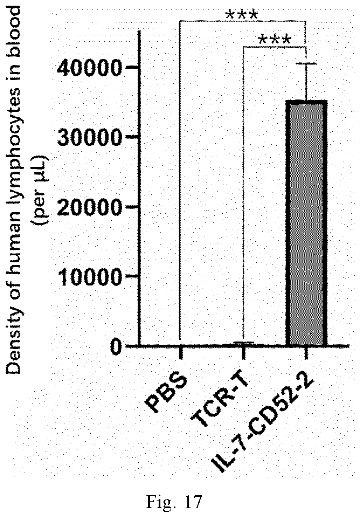

The experimental results were shown in Figure 14 to 17. Differences in tumor volume, tumor fluorescence value, and lymphocyte blood density between groups were statistically analyzed by unpaired two-tailed t-test. *: p<0.05; **: p<0.01; ***: p<0.001. Figure 14 shows that by the 21st day after administration, compared with the PBS-injected control group of tumor-bearing mice, the tumor volume increase of mice in the group administered with TCR-T cells was significantly inhibited. However, compared with the group administered with TCR-T, the tumor volume in the group administered with IL-7-CD52-2 TCR-T significantly became further smaller. Figure 15 shows consistency between the tumor fluorescence value and the trend in tumor volume change. 21 days after administration, the tumor fluorescence value of the group administered with TCR-T was significantly lower than that of the PBS group; while the fluorescence value of the group administered with IL-7-CD52-2 TCR-T was close to 0. Figure 16 shows that the fluorescence of A375-Luc2 tumor cells was basically non-visible in the mice in the group administered with IL-7-CD52-2 TCR-T. Figure 17 shows that 21 days after administration, the density of human lymphocytes in the mice from the group administered with TCR-T increased to a certain extent compared with the PBS group, while the density of human lymphocytes in the peripheral blood of mice from the group administered with IL-7-CD52-2 TCR-T increased dramatically compared with the TCR-T group.

The above results show that expressing IL-7-CD52-2 can significantly improve the tumor-killing effect of TCR-T cells in vivo.

Although specific embodiments of the present description have been described in detail, those skilled in the art will understand. Based on all the teachings that have been disclosed, various modifications and substitutions can be made to details, and these changes are all within the scope of the description. The full scope of the description is given by the appended claims and any equivalents thereof.

## Claims

1. A fusion protein comprising a functional domain and a GPI anchor region;
preferably, the functional domain functions through dimerization or multimerization.

2. The fusion protein of claim 1, wherein the GPI anchor region comprises one or more molecules selected from the groups consisting of: CD44, CD56, CD73, CD55, Thyl, AchE, IAP, ALPP, CD59, CD14, CD16, CD24, CD28, CD48, CD52, CD58, CD66a, CD66c, CD66d, CD66e, CD67, CD87, CD108, CD157, uPAR, JMH protein, GDNFR, CNTFR, TAG-1, PrP, phosphatidylinositol protein, semaphorin 7, CEA, GFR, Ly6G, transferrin receptor, contactin (F3) and T-cadherin, or GPI anchor domains thereof;
preferably, the GPI anchor region comprises one or more molecules selected from the group consisting of CD52, CD48, CD55, ALPP, and CD90 proteins, or GPI anchor domains thereof;
more preferably, the GPI anchor region comprises the sequence set forth in positions 232-268 of SEQ ID NO:2, or variants having at least 90% sequence identity thereto and being able to form GPI anchoring structures.

3. The fusion protein of claim 1 or 2, wherein the functional domain comprises:
an antibody or an antigen-binding fragment thereof, and/or an amino acid-based cytokine or a functional fragment thereof; preferably, the antibodies include blocking antibodies and activating antibodies, and the cytokines include interleukins or their receptors, chemokines or their receptors, interferons and TNF-α;
preferably, the fusion protein comprises one or more features selected from the group consisting of:
the antibody is an antibody targeting a tumor-associated antigen;
the interleukin or its receptor comprises one or more molecules selected from the group consisting of: IL-2, IL-4, IL-6, IL-7, IL-8, IL-10, IL-12, IL-15, CD25, IL-7R, IL-12Rβ1, IL-12Rβ2 and IL-15R;
The chemokine or its receptor comprises one or more molecules selected from the following: CXCL2, CXCL3, CXCL4, CXCL5, CXCL6, CXCL7, CXCL8, CXCL9, CXCL10, CXCL11, CXCL12, CXCL13, CXCL14, CCL1, CCL2, CCL3, CCL4, CCL5, CCL7, CCL8, CCL11, CCL13, CCL14, CCL15, CCL17, CCL18, CCL19, CCL20, CCL21, CCL22, CCL23, CCL24, CCL25, CCL26, CCL27, CCL28, CXCR1, CXCR2, CXCR3A, CXCR3B, CXCR4, CXCR5, CXCR6, CCR2, CCR5, CCR1, CCR3, CCR4 , CCR6, CCR7, CCR8, CCR9, CCR10 and XCR1; and
the interferon is IFN-γ.

4. The fusion protein of claim 1 or 2, wherein the fusion protein optionally further comprises a spacer located between the functional domain and the GPI anchor region;
preferably, the spacer comprises: a linker and/or a protein extracellular region; the protein extracellular region is, for example, a CD8 extracellular region and/or a BCMA extracellular region;
preferably,
the linkers comprise the sequence set forth in positions 177-188 of SEQ ID NO: 4, or a sequence that has at least 90% sequence identity thereto, and/or
the protein extracellular regions comprise the sequence shown in positions 177-231 of SEQ ID NO: 2 or positions 189-242 of SEQ ID NO: 4, or a sequence having at least 90% sequence identity thereto.

5. The fusion protein of claim 1 or 2, wherein the fusion protein further comprises a signal peptide;
preferably, the signal peptide is a signal peptide of the protein that the GPI anchor region is derived from;
more preferably, the signal peptide is a signal peptide of any one or more proteins selected from the group consisting of: CD52, CD48, CD55, ALPP, and CD90.

6. A nucleic acid molecule comprising a sequence selected from the group consisting of:
(1) the coding sequence of the fusion protein of any one of claims 1-6 or fragment thereof that functions as an amplification primer or detection probe,
(2) a variant having at least 80% sequence identity to (1),
(3) a complementary sequence of (1) or (2).

7. A nucleic acid construct, wherein the nucleic acid construct comprises the nucleic acid molecule of claim 6,
preferably, the nucleic acid construct is a cloning vector, an expression vector or an integration vector.

8. A cell, wherein the cell:
(1) containing, expressing and/or secreting the fusion protein of any one of claims 1-5, and/or
(2) containing any one or more of the nucleic acid molecules of claim 6 and the nucleic acid construct of claim 7,
preferably, the cell is a lymphocyte,
more preferably, the lymphocytes are T cells, NK cells or TILs;
more preferably, the T cells or TILs further express exogenous TCR and/or CAR.

9. A pharmaceutical composition, comprising pharmaceutically acceptable excipients, and any one or more selected from the group consisting of: the fusion protein of any one of claims 1-5, the nucleic acid molecule of claim 6, the nucleic acid construct of claim 7, and the cell of claim 8.

10. Use of the fusion protein of any one of claims 1 to 5, the nucleic acid molecule of claim 6, the nucleic acid construct of claim 7, the cell of claim 8, and the pharmaceutical composition of claim 9 in the manufacture of an antitumor medicament.
